# EUROPEAN PATENT SPECIFICATION

(11) **EP 0 981 308 B1**
(45) Date of publication and mention of the grant of the patent: **31.05.2006**
(21) Application number: 98922425.8
(22) Date of filing: 15.05.1998
(51) Int. Cl.: A61F 2/01, A61M 29/02

(54) **CATHETER-FILTER SET HAVING A COMPLIANT SEAL**
KATHETER UND FILTERANORDNUNG MIT EINER DRUCKAUSGEGLICHENEN DICHTUNG
ENSEMBLE CATHETER-FILTRE A ORGANE D'ETANCHEITE ELASTIQUE

(30) Priority: 16.05.1997 US 46777 P; 02.09.1997 US 57439 P
(43) Date of publication of application: 01.03.2000
(73) Proprietor: Gertler, Jonathan, Weston, MA 02193 (US); Kamm, Roger, Weston, MA 02193 (US)
(72) Inventor: Gertler, Jonathan, Weston, MA 02193 (US); Kamm, Roger, Weston, MA 02193 (US)
(74) Representative: Stevens, Ian Edward
(86) International application number: PCT/US1998/010220
(87) International publication number: WO 1998/051237

(56) References cited:
- WO-A-95/05209
- US-A- 4 723 549
- US-A- 5 053 008
- US-A- 5 470 314
- DATABASE WPI Week 8122 8 July 1981 Derwent Publications Ltd., London, GB; AN 78-70163A XP002075206 & SU 764 684 A (CHELY MED. INST.)

## Description

### Technical Field

The present invention relates to catheter-filter sets, including those for use in angioplasty and other procedures.

### Background Art

A large number of medical procedures utilize catheters. A catheter is defined herein and within the appended claims as a tubular, flexible instrument for insertion into a body cavity. Catheters may facilitate the withdrawal or introduction of fluids or other substances and may, in combination with other coupled components, perform a variety of other useful functions.

Catheters coupled with inflatable balloons provide the means to facilitate the unblocking of and the relief of constriction within various body passageways and vessels. Such angioplasty procedures can replace other more invasive surgical procedures and provide acceptable solutions to correct life threatening conditions. However, these procedures carry a risk of serious secondary problems associated with the transmission of unwanted material downstream of the operative site. Any material, such as plaque built up in arterial vessels, which does not adhere to the interior vas wall or is in another way removed from the vessel following treatment becomes a likely source of downstream blockage. In arterial angioplasty, embolic ischemic damage distal to the angioplasty site is a major complication of the procedure. Mobile arterial plaque is a major factor linked with ischemic stroke or end organ/limb infarction. In particular, carotid artery angioplasty is not a favored practice at present due to the risk of emboli and resulting stroke.

Some catheters coupled with downstream filtering capability have been previously disclosed. Several utilize wire mesh filters which are generally not compliant and do not accommodate localized changes in vessel diameter and shape which may be caused by on-going fluid flow restoration and pulsatility. Other designs contain deployment structures which may make insertion prior to and retraction after completion of a procedure problematic or unduly risky.

Typical prior art catheter arrangements are disclosed in United States patents 4,723,549; 4,794,928; 5,662,671; and 5,695,519. Document WO-A-95 05 209 discloses a catheter with filter according to the preamble of claim 1.

### Summary of the Invention

Various embodiments of the present invention solve problems of the prior art by providing radial compliance to accommodate localized changes in vessel diameter and shape which may be caused by on-going fluid flow restoration and pulsatility. Various embodiments of the invention avoid asperities or protrusions which can traumatize or otherwise damage or irritate interior vas walls. Similarly various embodiments provide for cushioning of the filter element against the vas wall when it is deployed. This cushioning effect is balanced with the requirement of effective sealing of the vas to prevent any unwanted downstream flow of matter.

Accordingly, in a first embodiment of the invention there is provided a catheter-filter set for use in a vas through which a biological fluid may flow. This embodiment includes a tubular member, having a lumen disposed along its length and an insertion end for insertion into the vas. The lumen defines a longitudinal axis and a radial direction perpendicular thereto. The embodiment also has a filter, coupled to the tubular member and having a circumference, for trapping undesired particles. (Unless the context otherwise requires, the term "particles" as used in this description and the accompanying claims refers to substances to be removed from a vas, and may include thrombotic material.) Finally, the embodiment includes a resilient member, having compliance in the radial direction, disposed circumferentially about the filter and, when deployed in the vas, forming a seal against the interior wall of the vas. The resilient member is a non-inflatable O-ring or sleeve. The outer surface of the resilient member may form the seal with the interior wall or may otherwise effectuate the seal. In yet another variation, the filter has a stowed position where the filter is radially confined and disposed in the lumen so that the catheter-filter set may be inserted into and removed from the vas, and a deployed position, where the filter is radially expanded.

Various embodiments of the present invention have a range of potential applications. The application of embodiments to angioplasty procedures wii be apparent to those skilled in the art. In addition, through either standard open technique or laparoscopic technique, retrieval of common duct stones in the biliary tree is facilitated by catheter passage utilizing embodiments herein. Similarly, through either standard open technique or cystographic technique, retrieval of ureteral and bladder duct stones is, also, facilitated by catheter passage via endoscopic or surgical methods. Embodiments herein provide improved devices to effectively retrieve such stones and to avoid their further passage or migration downstream. Embodiments herein may also employable as a temporary filtering device for the vena cava. In the process of lytic treatment for deep venous thrombosis, there is a risk of clot (thrombus) breaking loose and causing pulmonary embolism, a potentially fatal event. Although filters exist for vena cava use, these are permanent structures with attendant long term morbidity. Having an effective and retractable filter which is in place only for the time of significant risk, i.e., during deep venous clot lysis, would allow protection from pulmonary embolism and avoidance of the long term sequelae of a permanent filter insertion.

Discussion of medical procedures and associated devices in this description may focus, for example, upon arterial (blood circulation), biliary, and ureteral systems. This focus in no way limits the applicability of embodiments herein to any and all other uses for catheters with filtration capability known to those skilled in the art.

### Brief Description of the Drawings

Figs.1A and 1B are longitudinal views of a portion of a catheter-filter set, in the stowed and the deployed conditions, respectively, illustrating generally filter deployment according to an embodiment of the invention utilizing ribs for structuring the filter.
Figs.2A and 2B are longitudinal views of a catheter-filter set, in accordance with a tether deployment embodiment of the invention, showing stowed and deployed conditions, respectively.
Fig. 3 is a view of a catheter-filter set according to an embodiment of the invention utilizing a retractable O-ring.
Fig. 4 is a perspective view of a catheter-filter set according to an embodiment of the invention that is similar to the embodiment of Fig. 3, but utilizing a sleeve in lieu of the O-ring.
Fig. 5A is a perspective view of a catheter-filter set according to an embodiment of the invention utilizing a tethered O-ring, shown in the deployed condition. Fig. 5B is a longitudinal view of the same embodiment in the stowed condition.

### Detailed Description of Specific Embodiments

Various embodiments of the catheter-filter set described herein address a number of shortcomings inherent in previous designs. Some desirable features for the filter portion of the set are that it be easily confined in a radial direction for ease of set insertion and removal, that it be capable of capturing and safely removing all particles flowing downstream in a vas, and additionally, it should be designed so as to minimize the risk of accidental deployment and to collapse into position for removal in the event of failure. It should be atraumatic to the native vas wall and should accommodate changes in vas diameter.

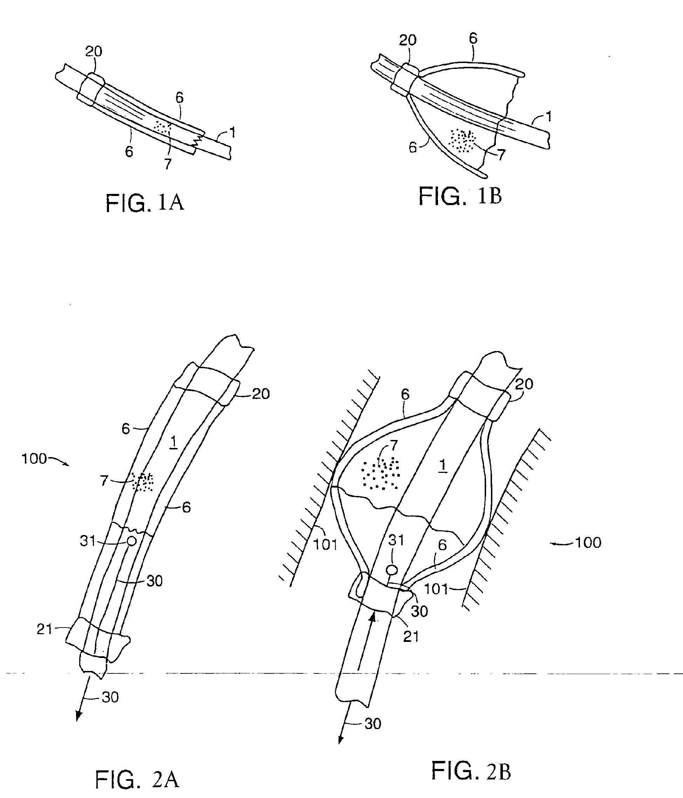

Figs. 1A and 1B are longitudinal views of a portion of a catheter-filter set, in the stowed and the deployed conditions, respectively, illustrating generally filter deployment according to an embodiment of the invention utilizing ribs 6 for structuring the filter **7.** Fig. 1A shows the ribs **6** and the filter **7** coupled to and juxtaposed with the catheter **1** in conjunction with the downstream collar **20.** In Fig. 1B the ribs **6,** which are resilient, are expanded radially from the catheter **1** to form a convex shape. The expanded ribs **6** support the filter **7** as shown. Preferably, the ribs **6** are free from asperities. The ribs **6** may be made of any resilient material with sufficient rigidity to support and to enable deployment of the filter **7.** The number of ribs **6** may be selected to be sufficiently large to facilitate a seal of the filter **7** with the interior wall of the vas **2** but not so numerous as to significantly obstruct fluid flow or convenient operation of the set. In the embodiment of Figs.1A and 1B, the ribs 6 are disposed radially outward from the filter **7.** In other embodiments of the invention, the ribs 6 may be disposed radially in ward from the filter **7,** so that, when stowed, the resilient ribs **6** lie immediately adjacent to the catheter **1.** Such other embodiments may deploy in a fashion analogous to opening of an umbrella. The ribs **6** may have one free end as in Figs. 1A and 1B, or be coupled to the catheter **1** at both ends and bow in the middle for deployment. The filter **7** may beneficially be made of a porous, compliant material in a manner known in the art. Suitable materials may include, but are not limited to, woven nylon, plastic resins such as PTFE sold under the Teflon trademark by Dupont of Wilmington, Delaware, other woven polymer, porous silicone rubber and latex rubber. Suitable materials are sufficiently porous to permit a small downstream flow of fluid yet capable of collecting any dangerous particles. For example, calculations suggest that, for an arterial application, a woven material with a fiber porosity of 90% and a spacing of 200 microns may pose little impediment to normal blood flow while trapping undesirable particles.

Figs.2A and 2B are longitudinal views of a catheter-filter set, in accordance with a tether deployment embodiment of the invention, showing stowed and deployed conditions, respectively. As shown in Fig. 2B, the ribs **6** of this embodiment are attached to the catheter 1 at both ends by collars **20** and **21.** The upstream end collar **21** is free to slide along the catheter **1** while the downstream end collar **20** is fixed. Collar **21** is tethered with line **30** so that an operator can, by applying an upstream force to line **30** slide collar **21** downstream toward collar **20.** This action urges the ribs 6 to form a convex shape and to expand radially from the catheter **1.** As Figs. 2A and 2B show, tethering may be accomplished by attaching one end of line **30** to collar **21,** feeding the line **30** through a port **31** provided by the catheter **1** at a position downstream from collar **21.** The line passes into a lumen of the catheter and exits the catheter **1** upstream at its retraction end. The ribs **6** may expand to the full radial extent of the vas **2.** Other material, as discussed in connection with other figures, is coupled to the ribs **6** proximate to their approximate midpoint in length to provide better sealing action and cushioning at the interface with the vas wall **101.** (For example, any suitable resilient sleeve or O-ring is utilized. ) The ribs **6** will return to the stowed position of Fig. 2A, in accordance with the embodiment of the invention, with the elimination of the applied upstream force on line **30.** This provides a set **100** which is biased to remain in and revert to the stowed position. Such bias provides for assured collapsibility upon retraction and offers a high degree of fail-safe operation by minimizing the chance of accidental deployment.

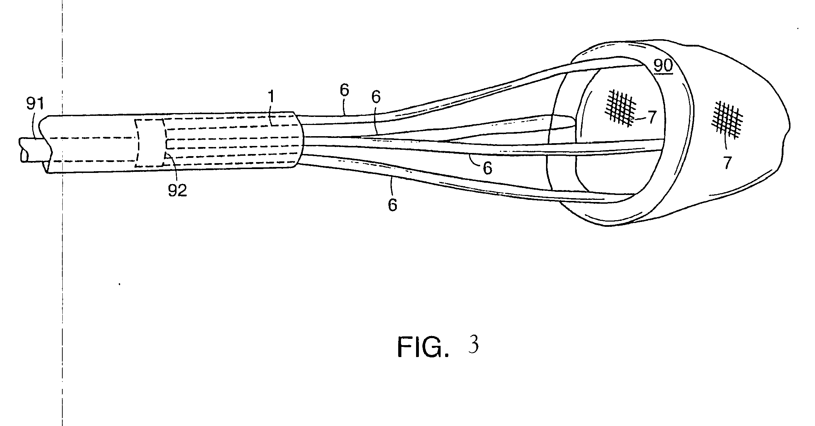

Fig. 3 is a perspective view of a catheter-filter set according to an embodiment of the invention utilizing a retractable O-ring. The downstream end of the catheter **1** is pictured at a location downstream of a suitable vas-modifying element. The filter **7** is fastened to an O-ring **90** which in turn, is coupled to a set of resilient ribs **6** (four of which are shown). When in the deployed position (shown) the ribs 6 are pushed out of the downstream end of the catheter **1.** The resilience of the O-ring **90** (optionally, in combination with shape memory of the ribs **6)** causes the ribs **6** to separate as the O-ring **90** deploys into its natural circular shape, producing a seal against the wall of the vas. In so doing, the O-ring **90** expands the filter **7** so that it can trap debris released from an upstream location. The filter **7** is returned to its original undeployed position by retracting the ribs **6** into the catheter **1,** exerting a radially-inward force on the O-ring **90,** causing it to buckle into a multi-lobed pattern with outside radial dimension much smaller than in the deployed position. The ribs **6** can be drawn into the catheter **1** by means of a stiff tether line **91** attached to the ribs **6** at their upstream end **92** within the catheter lumen. The tether line **91** must be sufficiently rigid that it can exert the force needed to deploy the filter **7** and the O-ring **90.** For ease of retrieval, the filter **7** can be sheathed by a second catheter that slides on the outer bore of the catheter **1,** slipping over the buckled O-ring **90** and filter **7.**

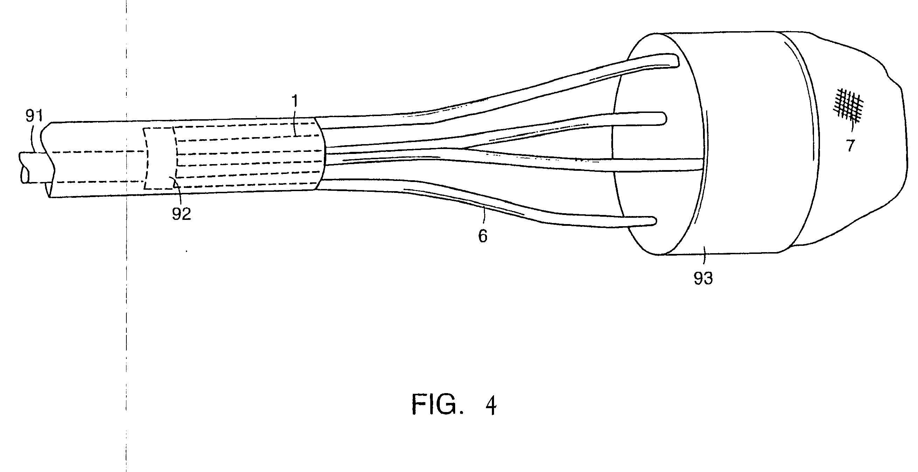

Fig. 4 is a perspective view of a catheter-filter set according to an embodiment of the invention that is similar to the embodiment of Fig. 3, but utilizing a cylindrical sleeve **93** in lieu of the O-ring **90.** An advantage of the sleeve is that the filter **7** can be entirely contained within the sleeve **93** when the ribs and filter are in the retracted position, thus eliminating the necessity for a second catheter.

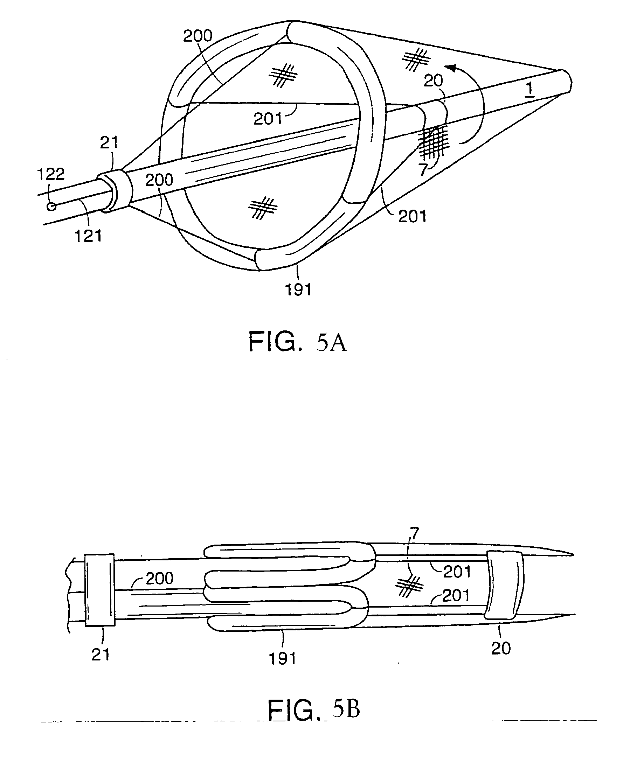

Fig. 5A is a perspective view of a catheter-filter set according to an embodiment of the invention utilizing a tethered O-ring, shown in the deployed condition. Fig. 5B is a longitudinal view of the same embodiment in the stowed condition. This embodiment shows that an O-ring **191** may be employed in situations in which the filter **7** is attached upstream of the end of the catheter **1.** Deployment, in this embodiment, is accomplished when the operator relaxes the force applied by the line **121** which passes through the wall of the catheter **1** at point **122.** The line **121** is attached to a collar **21** that can slide freely along the bore of the catheter **1.** When force is relaxed, the resilience of the O-ring **191** that exerts a tension in a plurality of tether lines **200** (two are shown) pulls the collar **21** in the downstream direction. The O-ring **191,** selected to be of an outer diameter when fully extended slightly larger than the normal diameter of the vas, provides a flexible seal against the wall of the vas when the tension force in tethers **200** is reduced. A filter **7** is attached around the circumference of the O-ring **191** and is deployed when the O-ring is allowed to expand to fill the vas. The O-ring **191** is also attached to a plurality of additional tethers **201** (two are shown) that are rigidly fixed to the downstream collar **20.** All tether lines **200** and **201** are inextensible and, in this embodiment, are attached at points distributed roughly equidistant around the circumference of the O-ring **191.** To retract and stow the filter, the operator pulls on the line **121** and the O-ring buckles into the configuration shown in Figure 5B due to the alternating attachments of the tether lines **200** and **201.** The buckling of the O-ring **191** also helps to gather together the filter **7.**

## Claims

1. A catheter-filter set (100) for use in a vas (2) through which a biological fluid may flow, the vas (2) having an interior wall (101), the fluid flow defining downstream and upstream directions, there being a risk of the presence of undesired particles in the fluid, the catheter-filter set (100) comprising:
a. a tubular member (1), having a lumen disposed along its length and an insertion end for insertion into the vas (2), the lumen defining a longitudinal axis and a radial direction perpendicular thereto;
b. a filter (7), coupled to the tubular member and having a circumference, for trapping undesired particles; **characterized in that** the catheter - filter set (100) further comprises
c. a resilient member, having compliance in the radial direction, disposed circumferentially about the filter (7) and, when deployed in the vas (2), forming a seal against the interior wall (101), wherein the resilient member is a non-inflatable O-ring (90, 191) or a non-inflatable sleeve (93).

2. A catheter-filter set (100) according to Claim 1, wherein the outer surface of the resilient member forms the seal with the interior wall (101).

3. A catheter-filter set (100) according to Claim 1, comprising ribs (6) for structuring the filter (7).

4. A catheter-filter set (100) according to Claim 4, wherein the ribs (6) have one free end or are coupled to the catheter (1) at both ends.

5. A catheter-filter set (100) according to Claim 3, wherein the ribs (6) are coupled to the catheter (1) at both ends by collars (20, 21), the upstream end collar (21) is free to slide along the catheter (1) while the downstream end collar (20) is fixed and collar (21) is tethered with line (30) so that an operator can, by applying an upstream force to line (30) slide collar (21) downstream toward collar (20), which action urges the ribs (6) to form a convex shape and to expand radially from the catheter (1), wherein the ribs (6) return to the stowed position with the elimination of the applied upstream force and the resilient O-ring (90, 191) or sleeve (93) is coupled to the ribs proximate to their approximate midpoint in length.

6. A catheter-filter set (100) according to Claim 1, wherein the filter (7) is fastened to the O-ring (90, 191) or cylindrical sleeve (93), which is coupled to a set of resilient ribs (6) such that in the deployed position the ribs (6) are pushed out of the downstream end of the catheter (1) and the resilience of the O-ring (90, 191) or sleeve (93) causes the ribs (6) to separate as the O-ring (90,191) or sleeve (93) deploys into its natural circular shape and produces a seal against the wall of the vas and the O-ring (90, 191) or sleeve (93) expands the filter (7) so that it can trap debris released from an upstream location.

7. A catheter-filter set (100) according to Claim 6, wherein the ribs (6) can be drawn into the catheter (1) by means of a stiff tether line (91) attached to the ribs (6) at their upstream end (92) within the catheter lumen.

8. A catheter-filter set (100) according to Claim 7, wherein the filter (7) is sheathed by a second catheter that slides on the outer bore of the catheter (1) slipping over the buckled O-ring (90, 191) or sleeve (93) and filter (7).

## Patentansprüche

1. Katheter-Filterset (100) zur Verwendung in einem Gefäß (2), durch welches ein biologisches Fluid strömen kann, wobei das Gefäß (2) eine Innenwand (101) besitzt und der Fluidstrom eine stromabwärts liegende und eine stromaufwärts liegende Richtung definiert, und die Gefahr besteht, dass unerwünschte Teilchen in dem Fluid vorhanden sind, wobei das Katheter-Filterset (100) folgende Bestandteile umfasst:
a. ein rohrförmiges Element (1), das ein in seiner Längsrichtung angeordnetes Lumen und ein Einführende zum Einführen in das Gefäß (2) aufweist, wobei das Lumen eine Längsachse und eine zu dieser senkrechte, radiale Richtung definiert,
b. ein Filter (7), das mit dem rohrförmigen Element gekoppelt ist und einen Umfang aufweist und dazu dient, unerwünschte Teilchen einzufangen,
**dadurch gekennzeichnet, dass** das Katheter-Filterset (100) weiterhin
c. ein elastisches Element umfasst, das in radialer Richtung nachgiebig ist und umfangsmäßig um das Filter (7) herum angeordnet ist, und das dann, wenn es in dem Gefäß (2) entfaltet ist, dicht an der Innenwand (101) anliegt, wobei das elastische Element ein nicht aufblasbarer O-Ring (90, 191) oder eine nicht aufblasbare Hülse (93) ist.

2. Katheter-Filterset (100) nach Anspruch 1, bei dem die äußere Oberfläche des elastischen Elementes dicht an der Innenwand (101) anliegt.

3. Katheter-Filterset (100) nach Anspruch 1, das Rippen (6) zum Strukturieren des Filters (7) umfasst.

4. Katheter-Filterset (100) nach Anspruch 3, bei dem die Rippen (6) ein freies Ende besitzen oder mit dem Katheter (1) an beiden Enden gekoppelt sind.

5. Katheter-Filterset (100) nach Anspruch 3, bei dem die Rippen (6) mit dem Katheter (1) an beiden Enden durch Krägen (20, 21) gekoppelt sind, wobei der am stromaufwärtigen Ende befindliche Kragen (21) frei längs des Katheters (1) gleiten kann, während der am stromabwärtigen Ende befindliche Kragen (20) festgelegt ist, und der Kragen (21) mit einer Schnur (30) so verbunden ist, dass eine Bedienungsperson **dadurch**, dass sie eine stromaufwärts gerichtete Kraft auf die Schnur (30) ausübt, den Kragen (21) stromabwärts zum Kragen (20) hin verschieben kann, wobei diese Einwirkung die Rippen (6) dazu zwingt, eine konvexe Form anzunehmen und sich vom Katheter (1) weg radial zu expandieren, wobei die Rippen (6) zu der zusammengefalteten Position beim Wegnehmen der angelegten, stromaufwärts gerichteten Kraft zurückkehren, und wobei der elastische O-Ring (90, 191) oder die Hülse (93) mit den Rippen in der Nähe ihrer ungefähren Mitte bezüglich der Länge verbunden ist.

6. Katheter-Filterset (100) nach Anspruch 1, bei dem der Filter (7) an dem O-Ring (90, 191) oder der zylindrischen Hülse (93) befestigt ist, der bzw. die mit einem Satz von elastischen Rippen (6) derart gekoppelt ist, dass in der entfalteten Stellung die Rippen (6) aus dem stromabwärts liegenden Ende des Katheters (1) herausgedrückt werden und wobei die Elastizität des O-Rings (90, 191) oder der Hülse (93) die Rippen (6) veranlasst, sich voneinander zu trennen, wenn sich der O-Ring (90, 191) oder die Hülse (93) in seine oder ihre natürliche, kreisförmige Form entfaltet und eine Dichtung gegen die Wand des Gefäßes erzeugt, und der O-Ring (90, 191) oder die Hülse (93) den Filter (6) expandiert, so dass er unerwünschte Teilchen einfangen kann, die an einer stromaufwärts liegenden Stelle freigesetzt werden.

7. Katheter-Filterset (100) nach Anspruch 6, bei dem die Rippen (6) in den Katheter (1) mit Hilfe einer steifen Anlein-Leitung (91) hineingezogen werden können, die an den Rippen (6) an ihrem stromaufwärts liegenden Ende (91) im Lumen des Katheters befestigt ist.

8. Katheter-Filterset (100) nach Anspruch 7, bei dem der Filter (7) durch einen zweiten Katheter ummantelt ist, der auf der äußeren Bohrung des Katheters (1) gleitet und über den vorgewölbten O-Ring (91, 191) oder die Hülse (93) und den Filter (7) schlüpft.

## Revendications

1. Ensemble (100) de filtre-cathéter destiné à être utilisé dans un capillaire (2) au travers duquel un liquide biologique peut s'écouler, le capillaire (2) ayant une paroi intérieure (101), l'écoulement de liquide définissant des directions en aval et en amont, un risque de la présence de particules non souhaitées dans le liquide existant, l'ensemble (100) de cathéter-filtre comprenant:
a. un élément tubulaire (1) ayant une lumière disposée le long de sa longueur et une extrémité d'insertion destinée à une insertion dans le capillaire (2), la lumière définissant un axe longitudinal et une direction radiale perpendiculaire à cette dernière ;
b. un filtre (7) couplé à l'élément tubulaire et ayant une circonférence, destiné à piéger des particules non souhaitées ; **caractérisé en ce que** l'ensemble (100) de cathéter-filtre comprend en outre
c. un élément résilient, ayant une élasticité dans la direction radiale, disposé de manière circonférentielle sur le filtre (7) et, lorsque déployé dans le capillaire (2), formant un joint contre la paroi intérieure (101), dans lequel l'élément résilient est un joint torique non gonflable (90, 191) ou un manchon non gonflable (93).

2. Ensemble (100) de cathéter-filtre selon la revendication 1, dans lequel la surface externe de l'élément résilient forme le joint avec la paroi intérieure (101).

3. Ensemble (100) de cathéter-filtre selon la revendication 1, comprenant des nervures (6) destinées à structurer le filtre (7).

4. Ensemble (100) de cathéter-filtre selon la revendication 4, dans lequel les nervures (6) ont une extrémité libre ou sont couplées sur le cathéter (1) au niveau des deux extrémités.

5. Ensemble (100) de cathéter-filtre selon la revendication 3, dans lequel les nervures (6) sont couplées au cathéter (1) au niveau des deux extrémités par des colliers (20, 21), le collier (21) d'extrémité en amont est libre de coulisser le long du cathéter (1) tandis que le collier (20) d'extrémité en aval est fixe et le collier (21) est retenu par un fil (30) de sorte qu'un opérateur peut, en appliquant une force en amont sur le fil (30) faire coulisser le collier (21) en aval vers le collier (20), laquelle action force les nervures (6) à former un profil convexe et à se dilater radialement à partir du cathéter (1), dans lequel les nervures (6) retournent vers la position rentrée avec l'élimination de l'application de la force en amont et le joint torique résilient (90, 91) ou le manchon (93) est couplé aux nervures à proximité de leur milieu approximatif en longueur.

6. Ensemble (100) de cathéter-filtre selon la revendication 1, dans lequel le filtre (7) est fixé sur le joint torique (90, 191) ou le manchon cylindrique (93), qui est couplé à un ensemble de nervures (6) résilientes de telle manière que dans la position déployée, les nervures (6) sont poussées hors de l'extrémité en aval du cathéter (1) et la résilience du joint torique (90, 191) ou du manchon (93) amène les nervures (6) à se séparer lorsque le joint torique (90, 191) ou le manchon (93) se déploie selon son profil circulaire naturel et produit un joint contre la paroi du capillaire et le joint torique (90, 191) ou le manchon (93) dilate le filtre (7) de sorte qu'il peut piéger des débris libérés d'un emplacement en amont.

7. Ensemble (100) de cathéter-filtre selon la revendication 6, dans lequel les nervures (6) peuvent être tirées dans le cathéter (1) au moyen d'un fil de retenue rigide (91) attaché sur les nervures (6) au niveau de leurs extrémités (92) en amont à l'intérieur de la lumière de cathéter.

8. Ensemble (100) de cathéter-filtre selon la revendication 7, dans lequel le filtre (7) est gainé par un second cathéter qui coulisse sur l'alésage externe du cathéter (1), glissant sur le joint torique déformé (90, 191) ou le manchon (93) et le filtre (7).
